# EUROPÄISCHE PATENTSCHRIFT

(11) **EP 2 968 261 B1**
(45) Veröffentlichungstag und Bekanntmachung des Hinweises auf die Patenterteilung: **05.08.2020**
(21) Anmeldenummer: 14786476.3
(22) Anmeldetag: 14.10.2014
(51) Int. Cl.: A61K 31/353, A61P 37/00, A61P 37/04

(54) **ZUSAMMENSETZUNG ZUR IMMUNMODULATION**
COMPOSITION FOR IMMUNE MODULATION
COMPOSITION DESTINEE A L'IMMUNOMODULATION

(30) Priorität: 16.10.2013 DE 102013017165
(43) Veröffentlichungstag der Anmeldung: 20.01.2016
(73) Patentinhaber: Eberhard Karls Universität Tübingen Medizinische Fakultät, 72074 Tübingen (DE)
(72) Erfinder: BUSCH, Christian, CH-8400 Winterthur (CH); VENTURELLI, Sascha, 79682 Todtmoos (DE)
(74) Vertreter: Witte, Weller & Partner Patentanwälte mbB
(86) Internationale Anmeldenummer: PCT/EP2014/071971
(87) Internationale Veröffentlichungsnummer: WO 2015/055623

(56) Entgegenhaltungen:
- EP-A1- 1 543 834
- EP-A1- 1 698 332
- WO-A2-02/39960
- DE-U1-202009 016 292
- US-A1- 2008 031 986
- MICHAEL PELUSO ET AL: "Xanthohumol and Related Prenylated Flavonoids Inhibit Inflammatory Cytokine Production in LPS-Activated THP-1 Monocytes: Structure-Activity Relationships and In Silico Binding to Myeloid Differentiation Protein-2 (MD-2)", PLANTA MEDICA, Bd. 76, Nr. 14, 22. März 2010 (2010-03-22) , Seiten 1536-1543, XP055154621, ISSN: 0032-0943, DOI: 10.1055/s-0029-1241013
- CHO Y C ET AL: "Xanthohumol inhibits IL-12 production and reduces chronic allergic contact dermatitis", INTERNATIONAL IMMUNOPHARMACOLOGY, ELSEVIER, AMSTERDAM, NL, Bd. 10, Nr. 5, 1. Mai 2010 (2010-05-01), Seiten 556-561, XP026996003, ISSN: 1567-5769 [gefunden am 2010-02-06]
- ZIH-YI WUN ET AL: "Anti-inflammatory effect of sophoraflavanone G isolated from Sophora flavescens in lipopolysaccharide-stimulated mouse macrophages", FOOD AND CHEMICAL TOXICOLOGY, Bd. 62, 2. September 2013 (2013-09-02), Seiten 255-261, XP055154596, ISSN: 0278-6915, DOI: 10.1016/j.fct.2013.08.072
- DONG WOOK KIM ET AL: "Effects of sophoraflavanone G, a prenylated flavonoid fro sophora flavescens on cyclooxygenase-2 and in vivo inflammatory response", ARCHIVES OF PHARMACAL RESEARCH, NATL. FISHERIES UNIVERSITY, PUSAN, KR, Bd. 25, Nr. 3, 1. Januar 2002 (2002-01-01) , Seiten 329-335, XP002975332, ISSN: 0253-6269
- ADRIANA ALBINI ET AL: "Mechanisms of the antiangiogenic activity by the hop flavonoid xanthohumol: NF- B and Akt as targets", THE FASEB JOURNAL, FEDERATION OF AMERICAN SOCIETIES FOR EXPERIMENTAL BIOLOGY, US, vol. 20, no. 3, 1 March 2006 (2006-03-01), pages 527-529, XP008144152, ISSN: 0892-6638, DOI: 10.1096/FJ.05-5128FJE

## Beschreibung

Die vorliegende Erfindung betrifft eine Zusammensetzung zur Immunmodulation sowie damit zusammenhängende Verwendungen und Verfahren.

Immuntherapien sind Behandlungsformen, bei denen das Immunsystem eines Organismus beeinflusst wird. Die Immunmodulation kann zur Dämpfung des Immunsystems, etwa nach Transplantationen und zur Vermeidung einer Abstoßungsreaktion dienen, oder auch zur Immunstimulation, also einer Erhöhung der natürlichen Immunreaktion. Die Wirkstoffe der Immuntherapie werden allgemein als Immunmodulatoren bezeichnet. Immunmodulatoren können demnach Immunstimulanzien oder Immuninhibitoren sein. Ein Beispiel für ein prominentes Immunstimulanz ist das Interferon, welches bei der Therapie von Hepatitis C-Erkrankungen eingesetzt wird. Auch pflanzliche Immunmodulatoren werden beschrieben, beispielsweise Extrakte aus der Hanfpflanze oder dem Roten Sonnenhut.

Für das maligne Melanom existieren bereits verschiedene in der Klinik eingesetzte immunmodulatorische Substanzen, die die Aktivität der natürlichen Killerzellen (NK-Zellen) modulieren. So kommt zu diesem Zwecke Interferon α in der adjuvanten Melanomtherapie in den Stadien II und III oder Interleukin 2 (IL-2) für die Behandlung des metastasierenden Melanoms im Stadium IV zum Einsatz.

Auch bei anderen Erkrankungen werden immunmodulatorische Substanzen einsetzt, zum Beispiel pegyliertes Interferon für die Behandlung von Hepatitis C oder Interferon β zur Behandlung der Multiplen Sklerose.

Die bisher eingesetzten immunmodulatorischen Substanzen haben sich in der Praxis meist nicht bewährt. Dies gilt insbesondere für die Melanomtherapie. Dort zeigen die bisher durchgeführten Immuntherapien in der Regel nur moderate Ansprechraten von kleiner 10 % mit gleichzeitig erheblichen Nebenwirkungen, wie zum Beispiel medikamentenbedingten Todesfällen bei systemischer IL-2-Therapie im Stadium IV. Auch im Bereich von Infektionen oder Autoimmunerkrankungen zeigen sich bei den derzeit zugelassenen Therapien erhebliche Schwierigkeiten. Trotz intensiver Immuntherapie sind chronische Verläufe der Regelfall, wie zum Beispiel bei Hepatitis C oder der Multiplen Sklerose.

Hinzu kommt, dass viele derzeit verwendete Immunmodulatoren teuer in der Herstellung sind. Sie bedingen aufwändige Synthesen und Reinigungsverfahren, so dass die Bereitstellung größerer Mengen letztlich auch für den Patienten größere Investitionen erfordern.

Im Stand der Technik wird eine inhibitorische Wirkung von 6- und 8-Prenylnaringenin auf die Zytokin- bzw. Prostaglandinproduktion von Entzündungszellen offenbart, z.B. Peluso et al., Planta Medica, Bd. 76, Nr. 14, 2010; Cho et al., International Immunopharmacology, Bd. 10, Nr. 5, 2010; WO 02/39960. Damit geht jedoch eine immuninhibierende Wirkung einher.

Vor diesem Hintergrund ist es eine Aufgabe der vorliegenden Erfindung, einen neuen Immunmodulator bereitzustellen, mit dem die im Stand der Technik beschriebenen Probleme vermieden oder zumindest vermindert werden.

Diese Aufgabe wird durch die Bereitstellung einer Zusammensetzung zur Verwendung in der Immunstimulation gelöst, die als Wirkstoff 6- und/oder 8- Prenylnaringenin (PN) aufweist.

Prenylflavonoide gehören zu der Gruppe der Flavonoiden, einer großen Gruppe von niedermolekulargewichtigen polyphenolischen Verbindungen. Flavonoide werden in Pflanzen gefunden und bestehen aus Flavonen, Flavonolen, Flavononen, Flavan-3-olen und Anthocyaninen. Diese sekundären Pflanzenmetabolite spielen eine Rolle in der Abwehr der Pflanze gegen Mikroorganismen oder Pilzen und dem Schutz vor oxidativem Stress.

Ein wichtiger Vertreter der Prenylflavonoide ist das Prenylnaringenin (PN).

Die Erfinder konnten in der Zellkultur eindrucksvoll aufzeigen, dass 6 und/oder 8-Prenylnaringenin eine Stimulation des Immunsystems bewirkt.

Bei dem Prenylnaringenin und den spezifischen hierunter fallenden erfindungsgemäßen Verbindungen kann es sich um den bzw. die alleinigen Wirkstoffe i.S.e. Monotherapie handeln. Es können jedoch ggf. weitere Wirkstoffe vorgesehen werden, so dass sich ggf. synergistische Wirkungen ergeben.

Die der Erfindung zugrundeliegende Aufgabe wird hiermit vollkommen gelöst.

Die Zusammensetzung weist 6-Prenzylnaringenin (6-PN) und/oder 8-Prenylnaringenin (8-PN) auf.

6-PN und 8-PN sind sogenannte Prenylflavonoide. Diese finden sich in niedrigen Konzentrationen beispielsweise im Hopfen und in Bier. Die Struktur dieser Klasse von Flavonoiden leitet sich vom 2-Phenylchrom-4-on ab. 6-PN und 8-PN sind Isomere. Der Unterschied liegt in der Position der aus fünf Kohlenstoffatomen bestehenden Prenylgruppe. 6-PN und 8-PN sind Produkte einer Ringschlussreaktion eines gemeinsamen Vorläufermoleküls, Desmethylxanthohumol, einem Polyphenol, das auf der Struktur von 1,3-Diphenyl-2-propen-1-on basiert. Beide Moleküle existieren als Enantiomere (R,S).

8-PN weist eine starke Bindeaffinität zu Östrogenrezeptoren des Rattenuterus auf und ist als potentes Phytoestrogen aufgrund einer charakteristischen Beabstandung der Hydroxylgruppen identifiziert worden, die Beta-Estradiol imitieren; vgl. Milligan et al. (1999), Identification of potent phytoestrogen in hops (Humulus lupulus L.) and beer, J. Clin. Endocrinol. Metab. 84, 2249-2252.

Neben den antioxidativen Eigenschaften zeigen einige Flavonoide auch Antikrebseigenschaften; vgl. Hodek et al. (2002) Flavonoids-potent and versatile biologically active compounds interacting with cytochromes p. 450, Chem. Biol. Interact. 139, 1-21. 8-PN inhibiert die Angiogenese, induziert durch den basischen Fibroblastenwachstumsfaktor und vaskulären endothelialen Wachstumsfaktor, in einem dreidimensionalen Collagengel in vitro und in Chorioallantoismembran assays in vivo; vgl. Hepper et al. (2004), 8-Prenylnaringenin, A novel phytoestogen, inhibits angiogenesis in vitro and in vivo, J. Cell Physiol. 199, 98-107. 8-PN imitiert die Wirkungen von 17β-Estradiol auf den Brustkrebszellen MCF-7; vgl. Rrong et al. (2001), 8-Prenylnaringenin, the phytoestrogen in hops and beer, upregulates the function of the e-cadherin-/catenin complex in human mammary carcinoma cells, Eur. J. Cell Biol. 80, 5 180-585. 8-PN induziert ferner die Apoptose in MCF7-Zellen und in einer gegen eine Vielzahl von Wirkstoffen resistenten Leukämieblaste; vgl. Brunelli et al. (2007), 8-Prenylnaringenin, inhibits estrogen receptor-α-mediated cell growth and induces apoptosis in MCF-7 breast cancer cells, J. Steroid. Biochem. Mol. Biol. 107, 140-148, und Diller et al. (2007), Ability of prenylflavanones present in hops to induce apoptosis in a human Burkitt lymphoma cell line, Planta Med. 73, 755-761. Vor Kurzem wurde ferner die Inhibition des P-Glycoproteins, dem mit einer Multiresistenz assoziierten Transporterprotein, und die Inhibition von MRP1 durch 8-PN beschrieben; vgl. Wesolowska et al. (2010), 8-Prenylnaringenin is an inhibitor of multidrug resistance-associated transporters, P-glycoprotein and MRP1, Eur. J. Pharmacol. 644, 32-40. Ferner inhibiert 8-PN direkt die Aktivierung des PI (3) K/Akt-Signalweges in MCF-7-Zellen in vitro; Brunelli et al. (2009), 8-Prenylnaringenin inhibits epidermal growth factorinduced MCF-7 breast cancer cell proliferation by targeting phosphatidylinositol-3-OH kinase activity, J. Steroid. Biochem. Mol. Biol. 113, 163-170. 6-PN und 8-PN zeigen antiproliferative Wirkungen auf die humanen Prostatakrebszelllinien PC-3 und DU145 in vitro; Delmulle et al. (2006), Anti-proliferative properties of prenylated flavonoids from hops (Humulus lupulus L.) in human prostate cancer cell lines, Phytomedicine 13, 732-734. Dies erfolgt in Abwesenheit einer Caspase-3-Aktivierung und typischen apoptotischen morphologischen Merkmalen; Delmulle et al. (2008), Treatment of PC-3 and DU145 prostate cancer cells by prenylflavonoids from hop (Humulus lupulus L.) induces a caspase-independent form of cell death, Phytother. Res. 22, 197-203.

In der deutschen Patentanmeldung DE 10 2013 104 342 wird die Verwendung von 6-PN und 8-PN zur Behandlung eines Melanoms und einer Melanomvorstufe der Haut sowie einer Schleimhautmetastase beschrieben. Dort wird außerdem beschrieben, dass es sich bei 6-PN und 8-PN um distinkte Inhibitoren von Histondeacetylasen (HDACs) der Klassen I, II und IV handelt.

Die Eignung von 6-PN und 8-PN als Immunstimulator ist im Stand der Technik bisher nicht beschrieben.

Die Erfinder konnten eindrucksvoll demonstrieren, dass 6-PN und 8-PN immunstimulatorische Wirkungen auf das angeborene Immunsystem bzw. auf natürliche Killerzellen (NK-Zellen) ausüben. Die Prenylnaringenine erhöhen massiv die Viabilität von NK-Zellen, verlängern das Gesamtüberleben von NK-Zellen und bewirken ein gesteigertes "Killing" von veränderten Zellen, wie Virus-infizierten oder Tumorzellen. Die Prenylnaringenine aktivieren demnach direkt oder indirekt die NK-Zellen.

Die Erfindung betrifft deshalb eine Immunstimulation.

Diese Maßnahme hat den Vorteil, dass über eine gezielte Aktivierung des angeborenen Immunsystems eine Stimulation der endogenen Abwehrmechanismen erfolgt und der Organismus dadurch verbessert gegen Krankheiten geschützt ist.

Nach einer bevorzugten Weiterentwicklung handelt es sich bei der immunstimulation um eine solche des angeborenen Immunsystems.

Diese Maßnahme hat den Vorteil, dass zielgerichtet auf den Teil des Immunsystems positiv eingewirkt wird, über den ein Großteil von Infektionen und Erkrankungen abgewehrt werden kann.

Nach einer erfindungsgemäßen Weiterentwicklung bewirkt die Immunstimulation eine Stimulation und/oder Erhöhung der Viabilität und/oder der Erhöhung der Lebensdauer und/oder Erhöhung der Aktivität der natürlichen Killerzellen (NK-Zellen).

Diese Maßnahme hat den Vorteil, dass auf den zentralen Zelltyp des angeborenen Immunsystems stimulierend eingewirkt wird. Der Körper wird dadurch gestärkt und ist in der Lage, verbessert abnormale Zellen, wie Tumorzellen oder Virusinfizierte Zellen, zu erkennen und abzutöten. Dabei kann die Aktivierung der NK-Zellen sowohl durch die direkte Aktivierung der NK-Zellen erfolgen, z.B. durch Hochregulation stimulatorischer Mechanismen. So können spezifische Oberflächenliganden bzw. Rezeptoren auf Zielzellen, bspw. Tumor- oder virusinfizierten Zellen, hochreguliert werden, welche durch Zellen des angeborenen Immunsystems erkannt werden und somit zu einer gesteigerten Aktivität führen. Ferner kann die Aktivierung der NK-Zellen durch indirekte Aktivierung erfolgen, z.B. durch Herunterregulierung inhibitorischer Mechanismen.

Bei der erfindungsgemäßen Zusammensetzung handelt es sich vorzugsweise um eine pharmazeutische Zusammensetzung, die weiter vorzugsweise zur Behandlung einer Krankheit ausgestaltet ist, die ausgewählt ist aus der Gruppe bestehend aus viralen Infektionen, Autoimmunerkrankungen oder Tumorerkrankungen.

Es versteht sich, dass die erfindungsgemäße pharmazeutische Zusammensetzung eine pharmazeutisch akzeptable Formulierung aufweisen kann. Pharmazeutisch akzeptable Formulierungen sind im Stand der Technik hinreichend bekannt. Beispielhaft wird auf die Abhandlung von Kibbe A. (2000), Handbook of Pharmaceutical Excipients, 3. Auflage, American Pharmaceutical Association and Pharmaceutical Press, verwiesen. Die erfindungsgemäße pharmazeutische Zusammensetzung kann ferner Zusätze enthalten. Diese umfassen jedwede Verbindung oder Zusammensetzung, die für die erfindungsgemäße Verwendung vorteilhaft sind, worunter Salze, Bindemittel, Lösungsmittel, Dispersionsmittel und weitere in Zusammenhang mit der Formulierung von Arzneimittel üblicherweise verwendete Stoffe fallen.

Die erfindungsgemäße Zusammensetzung kann in einer Applikationsform vorliegen, die eine systemische oder eine topische Applikation ermöglicht, wie beispielsweise als Injektions- oder Trinklösung, als Salbe, Creme, Lotion, Gel, Paste, transdermales therapeutisches System, Schaum, Puder. Der Wirkstoff kann außerdem verkapselt, beispielsweise in Liposomen, vorliegen.

Nach einer alternativen Ausführungsform handelt es sich bei der erfindungsgemäßen Zusammensetzung um ein Lebensmittelprodukt.

Diese Maßnahme hat den Vorteil, dass die von den Erfindern erkannten positiven Eigenschaften der Prenylflavonoide über die Nahrung aufgenommen werden können. Die Bereitstellung eines aufwändig synthetisierten und zuzulassenden Arzneimittels ist nicht erforderlich. Das Lebensmittelprodukt kann das Prenylflavonoid bzw. 6-PN und/oder 8-PN in natürlich vorkommender Konzentration enthalten. Das Lebensmittelprodukt kann jedoch auch bezüglich dieser Wirkstoffe angereichert sein, so dass sich ein erhöhter positiver Effekt auf die Gesundheit des Organismus ergibt. Das Lebensmittelprodukt kann sowohl eine Flüssigkeit als auch ein festes Nahrungsmittel sein.

Dabei ist es bevorzugt, wenn es sich bei dem Lebensmittelprodukt um ein solches auf Hopfenbasis handelt.

Diese Maßnahme hat den Vorteil, dass ein natürlicher "Rohstoff" für die Prenylflavonoide bzw. 6-PN und/oder 8-PN verwendet wird, der in großen Mengen verfügbar ist und aus dem sich die Wirkstoffe gut isolieren bzw. anreichern lassen.

Dabei ist es bevorzugt, wenn in der erfindungsgemäßen Zusammensetzung der Wirkstoff, d.h. das Prenylflavonoid bzw. Prenylnaringenin (PN), vorzugsweise 6-PN und/oder 8-PN, in micellierter Form bzw. verpackt in Micellen vorliegt.

Micellen, auch Assoziationskolloide genannt, sind Aggregate aus amphiphilen Molekülen bzw. grenzflächenaktiven Substanzen, die sich in einem Dispersionsmedium, bspw. Wasser, spontan zusammenlagern. Sie weisen gegenüber Liposomen einen deutlich geringeren Durchmesser auf. Dieser liegt durchschnittlich im Nanometerbereich, zwischen ca. 1 nm- ca. 100 nm, vorzugsweise zwischen ca. 20 nm - 80 nm, weiter vorzugsweise ca. 35 nm - 65 nm, höchst vorzugsweise ca. 30 nm oder 50 nm. "Ca." bedeutet in diesem Zusammenhang ± 10%. Die Verpackung des Wirkstoffs in Micellen hat den Vorteil, dass dadurch die Bioverfügbarkeit im Organismus deutlich erhöht wird. Die Micellen, die den Wirkstoff enthalten, liegen in einem Solubilisat vor. Der Wirkstoff ist sozusagen "solubilisiert". Als besonders geeignet hat sich eine Verpackung in Micellen erwiesen, die von der Fa. Aquanova, Darmstadt, Deutschland, unter der Bezeichnung "Produkt-Micelle" angeboten werden. Die erhaltenen Solubilisate werden unter der Bezeichnung NovaSOL® geführt. Die Produkt-Micellen sind thermisch, mechanisch und auch in der Magensäure stabil und weisen durchschnittlich einen Durchmesser von nur ca. 30 nm auf.

Ein weiterer Gegenstand der vorliegenden Offenbarung betrifft die Verwendung eines Prenylflavonoids, vorzugsweise von Prenylnaringenin, weiter vorzugsweise von 6- und/oder 8-Prenylnaringenin, als Immunmodulator, vorzugsweise Immunstimulator, weiter vorzugsweise zur Stimulation und/oder Erhöhung der Viabilität und/oder der Erhöhung der Lebensdauer der natürlichen Killerzellen (NK-Zellen).

Ein weiterer Gegenstand der vorliegenden Offenbarung betrifft ein Verfahren zur Immunmodulation, vorzugsweise zur Immunstimulation, weiter vorzugsweise zur Stimulation und/oder Erhöhung der Viabilität und/oder der Erhöhung der Lebensdauer und/oder Erhöhung der Aktivität der natürlichen Killerzellen (NK-Zellen) bei einem Lebewesen, vorzugsweise einem Menschen, das folgende Schritte aufweist: (1) Bereitstellung der erfindungsgemäßen Zusammensetzung, und (2) Applikation der Zusammensetzung in bzw. an das Lebewesen, und (3) gegebenenfalls Wiederholung der Schritte (1) und (2), wobei vorzugsweise die Applikation systemisch und/oder topisch erfolgt.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nun anhand von Ausführungsbeispielen näher erläutert, aus denen sich weitere Eigenschaften, Merkmale und Vorteile ergeben. Die Ausführungsbeispiele sollen die Erfindung illustrieren, schränken jedoch deren Reichweite nicht ein. Dabei wird Bezug genommen auf die beigefügten Figuren, in denen Folgendes dargestellt ist:

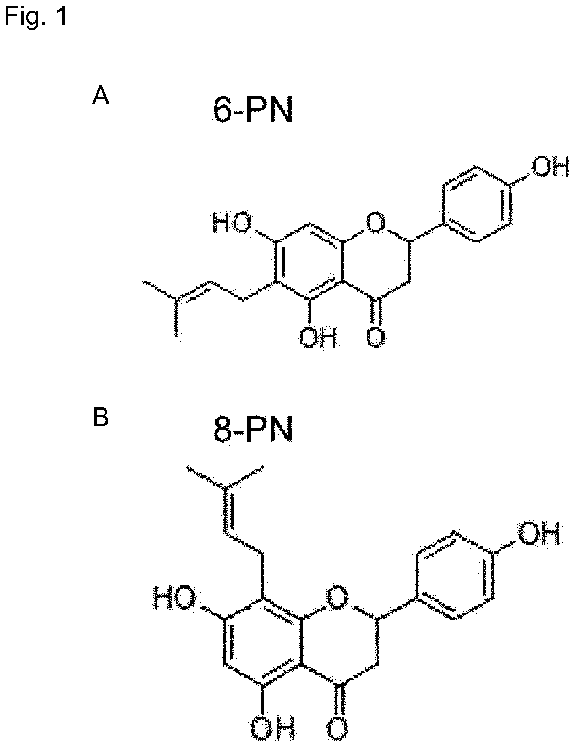

- Fig. 1: Chemische Strukturen von 6-PN und 8-PN. Dargestellt sind die chemischen Strukturen von 6-Prenylnaringenin (5,6-Dihydroxy-2-(4-Hydroxy-Phenyl)-6-(3-Methyl-But-2-enyl)-Chroman-4-on) und 8-Prenylnaringenin (5,7-Dihydroxy-2-(4-Hydroxy-Phenyl)-8-(3-Methyl-But-2-enyl)-Chroman4-on).
- Fig. 2: Viabilitätsmessung an NK-Zellen nach 48 Stunden Behandlung mit verschiedenen Konzentrationen von 6-PN oder 8-PN.
- Fig. 3: Messung des gesamten Überlebens von NK-Zellen nach einmaliger Behandlung mit verschiedenen Konzentrationen von 6-PN oder 8-PN.
- Fig. 4: Analyse der Aktivität von NK-Zellen auf humanen HepG2-Tumorzellen.
- Fig. 5: 6-PN und 8-PN aktivieren primäre humane Immunzellen und reduzieren die Tumormasse eines metastasierten malignen Melanoms.
- Fig. 6: 6-PN und 8-PN erhöhen/verlängern die Viabilität von NK-Zellen von gesunden Spendern, gemessen in einem automatisierten Viabilitäts-/Zellzähl-Gerät.
- Fig. 7: 6-PN und 8-PN erhöhen/verlängern die Viabilität von PBMCs von gesunden Spendern, gemessen in einem automatisierten Viabilitäts-/Zellzähl-Gerät.
- Fig. 8: 6-PN und 8-PN erhöhen die metabolische Aktivität von PBMCs von gesunden Spendern oder der standardisierten, käuflichen NK-Zelllinie NKL, gemessen mit dem Promega CellTiter-Blue Assay.

### Ausführungsbeispiele

### 1. Synthese von 6-Prenylnaringenin und 8-Prenylnaringenin (PN)

8-Prenylnaringenin und 6-Prenylnaringenin wurden gemäß Gester et al. (2001), An efficient synthesis of the potent phytoestrogens 8-prenylnaringenin und 6-(1,1-dimethylallyl)naringenin by europium (III)-catalyzed Claisen rearrangement, Tetrahedron 57, 1015-1018 bzw. Tischer und Metz (2007), Selective C-6 prenylation of flavonoids via europium(III)-catalyzed Claisen rearrangement and cross-metathesis, Advanced Synthesis & Catalysis 349, 147-151, synthetisiert. Der Inhalt der beiden vorstehenden Publikationen ist durch Inbezugnahme Bestandteil der vorliegenden Anmeldung.

### 2. Erhöhte Viabilität von humanen NK-Zellen durch 6-PN und 8-PN

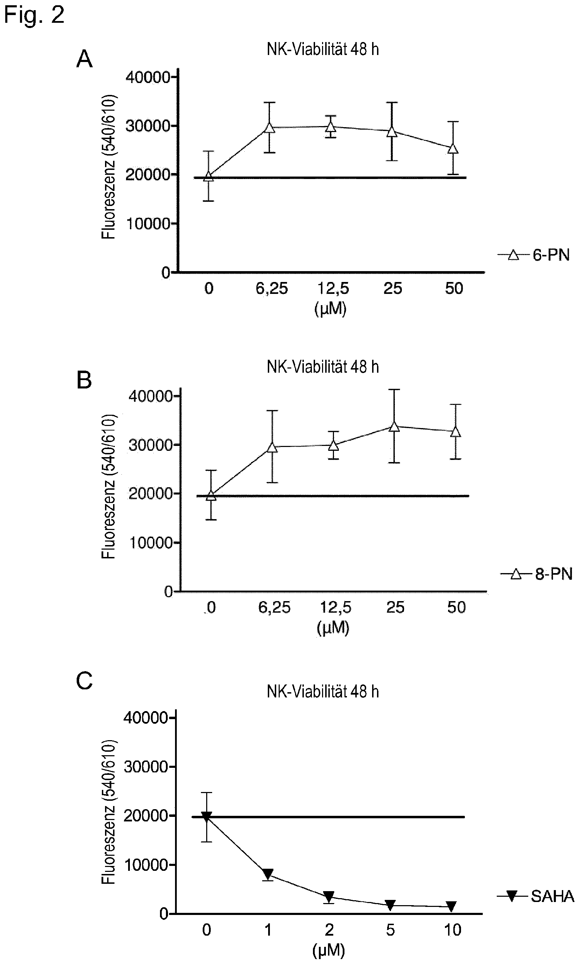

Humane NK-Zellen wurden 48 Stunden mit 6,25, 12,5, 25 oder 50 µM von 6-PN oder 8-PN behandelt. Anschließend wurde eine Viabilitätsmessung durchgeführt (CellTiterBlue Viability Assay, Promega, Madison, Vereinigte Staaten von Amerika). Als Referenzfarbstoff für die beiden natürlichen HDACi 6-PN und 8-PN wurde der klinisch eingesetzte und etablierte HDACi SAHA (Vorinostat) in geringen Konzentrationen verwendet. Das Ergebnis ist in der Fig. 2 wiedergegeben. Dargestellt sind die Mittelwerte mit Standardabweichung aus drei unabhängigen Experimenten mit expandierten NK-Zellen von drei gesunden humanen Spendern. Die waagerechte durchgezogene Linie visualisiert den Wert der unbehandelten Kontrolle; ein Abfall des Fluoreszenzsignals bedeutet Reduktion der Viabilität. Dabei zeigt sich, dass in Konzentrationsbereichen zwischen 6,25 und 50 µM 6-PN oder 8-PN (Fig. 2A, B) eine zum Teil deutliche Erhöhung der Viabilität von humanen NK-Zellen gegenüber dem Kontrollansatz sowie den mit SAHA behandelten Zellen messbar ist. Die SAHA-Behandlungsgruppe zeigt hingegen über den gesamten Konzentrationsbereich eine deutliche Abnahme der Viabilität der NK-Zellen (Fig. 2C).

### 3. Verlängertes Gesamtüberleben von humanen NK-Zellen

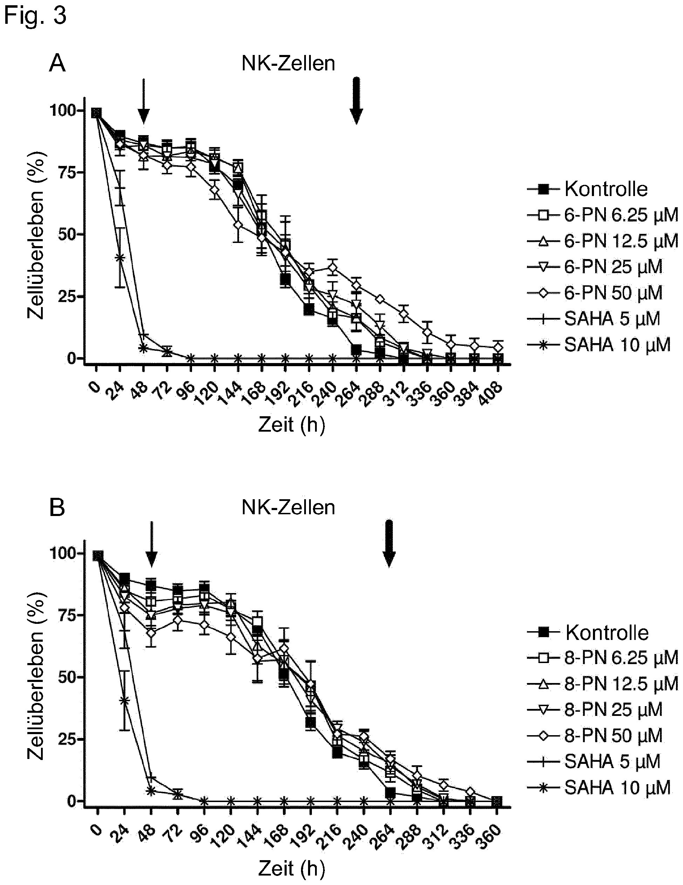

Humane NK-Zellen wurden mit 6,25, 12,5, 25 oder 50 µM von 6-PN oder 8-PN behandelt. Anschließend wurde über Trypanblau-Färbung und tägliche mikroskopische Auszählung in standardisierten C-Chip-Zählkammern das Gesamtüberleben der NK-Zellen gemessen. Als Referenzwirkstoff wurde erneut der HDACi SAHA (Vorinostat) verwendet. Das Ergebnis ist in der Fig. 3 dargestellt. Dargestellt ist der Mittelwert mit Standardabweichung aus drei unabhängigen Experimenten mit expandierten NK-Zellen von drei gesunden humanen Spendern. Der linke Pfeil visualisiert < 10 % lebende Zellen in der SAHA-Behandlungsgruppe; der rechte Pfeil visualisiert < 10 % lebende Zellen in der unbehandelten Kontrollgruppe. Dabei zeigt sich, dass die Überlebensrate der NK-Zellen über sämtliche der getesteten Konzentrationen von 6-PN (Fig. 3A) und 8-PN (Fig. 3B) hinweg gegenüber der unbehandelten Kontrolle und den mit SAHA behandelten Zellen deutlich erhöht wird.

### 4. Gesteigerte Aktivität von humanen NK-Zellen auf humanen Tumorzellen

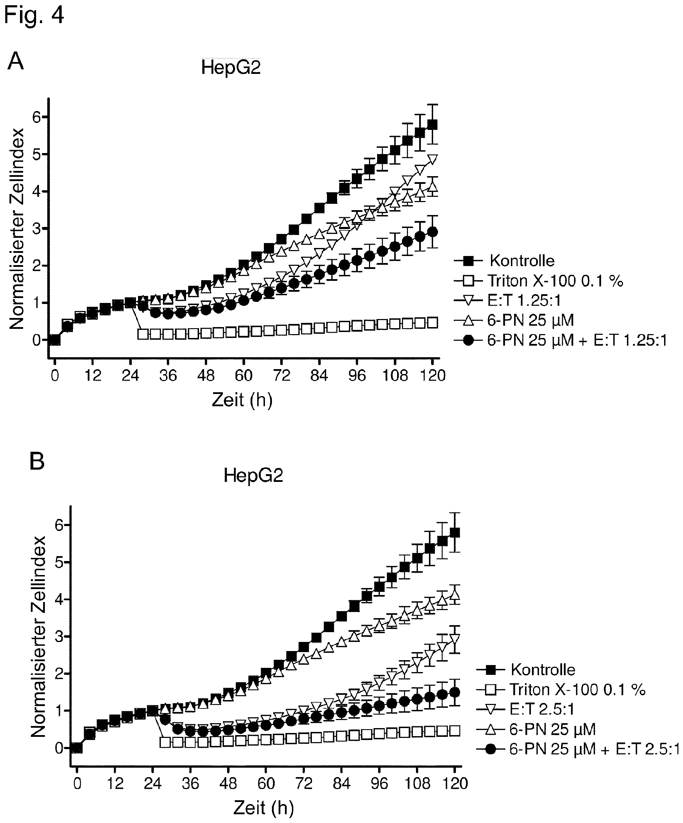

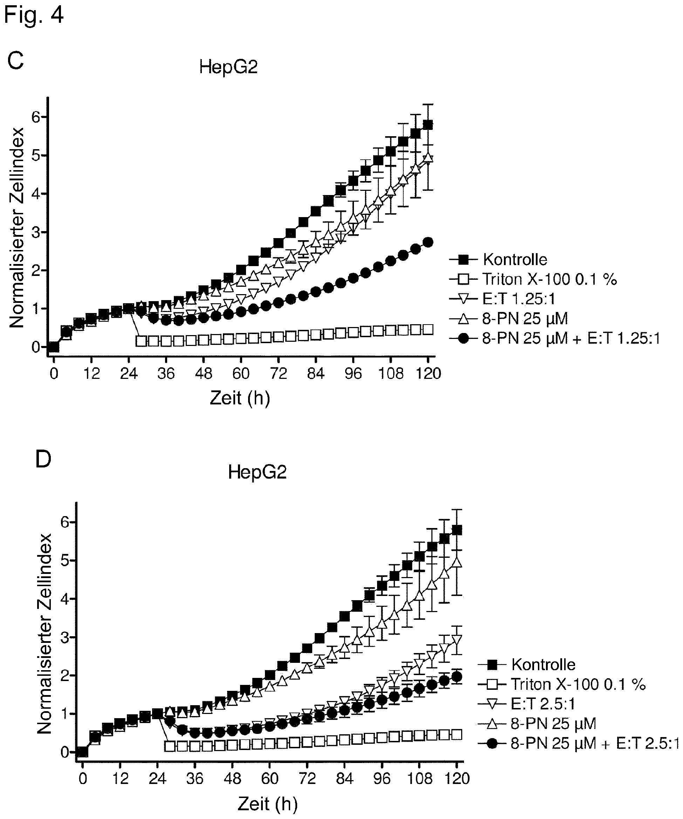

In einem weiteren Experiment wurde die NK-Aktivität auf humanen HepG2-Tumorzellen mittels Real-time-Cell-Monitoring (Roche Applied Science xCELLigence SP-System, Mannheim, Deutschland) analysiert. HepG2-Tumorzellen zeichnen sich durch eine geringe Sensitivität gegenüber 6-PN bzw. 8-PN aus. Gemessen wurde die zelluläre Impedanz der adhärenten Tumorzellen als Parameter für den Zellstatus (Zellzahl, Viabilität, Morphologie, Adhäsion). Als Positivkontrolle für die Induktion von Zelltod wurde Triton X-100 verwendet. Es wurden drei unabhängige Experimente mit expandierten NK-Zellen von drei gesunden humanen Spendern durchgeführt. Das Ergebnis ist in der Fig. 4 gezeigt. Dargestellt sind die Mittelwerte mit Standardabweichung von einem exemplarischen Versuch. Die HepG2-Tumorzellen (Target) wurden ab dem Behandlungsstart (24 Stunden) mit 25 µM von 6-PN oder 8-PN bzw. mit NK-Zellen (Effektor) inkubiert (Effektor zu Target-Verhältnis (E:T) = 1,25:1 oder 2,5:1) oder mit der Kombination aus HDACi und NK-Zellen. Die Normalisierung erfolgte zum Behandlungsstart und ein Abfall der Impedanz (normalisierter Zellindex) bedeutet Reduktion der Zellviabilität bzw. des Zellstatus. Dabei zeigt sich im Vergleich zur Kontrolle für den induzierten Zelltod (Triton X-100), dass es sowohl bei einer Behandlung mit 6-PN (Fig. 4A, B) als auch 8-PN (Fig. 4C, D) zu einer gesteigerten Aktivität der humanen NK-Zellen gegenüber humanen Tumorzellen kommt. Versuche mit anderen Konzentrationen von 6-PN oder 8-PN (z.B. 6,25 µM) oder einem anderen E:T-Verhältnis (z.B. 5:1) sowie einer weiteren Tumorzelllinie (z.B. Hep3B) ergaben äquivalente Ergebnisse.

### 5. Bestätigung der antitumoralen Immunmodulation mittels primärem Patientenmaterial

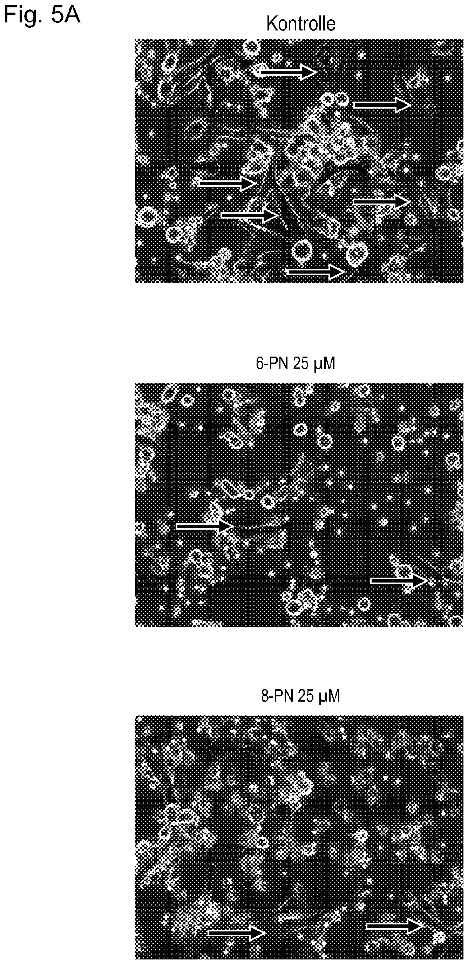

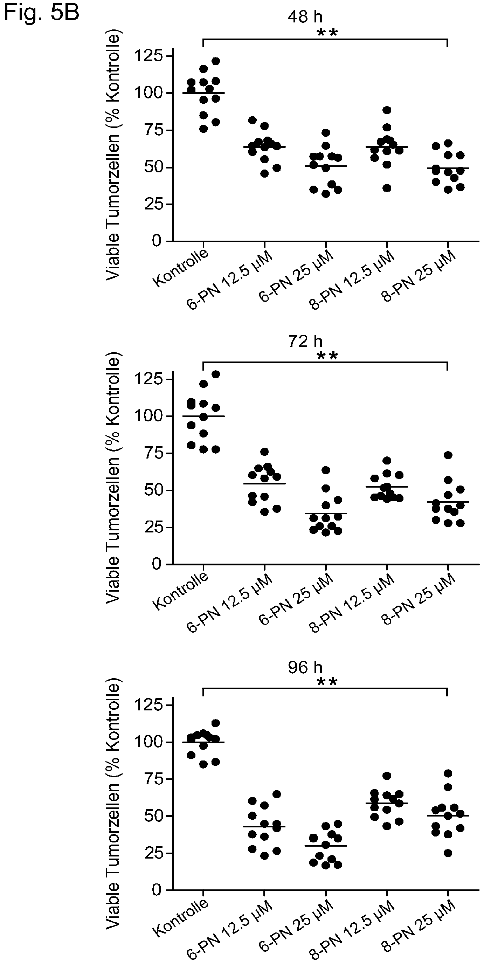

In einem weiteren Experiment an primärem Patientenmaterial wurde demonstriert, dass 6-PN und 8-PN primäre humane Immunzellen aktivieren und die Tumormasse eines metastasierten malignen Melanoms reduzieren. Das Ergebnis dieses Experiments ist in der Fig. 5 gezeigt: **A** Mikroskopische Aufnahmen in 200x Vergrößerung, welche die Melanom-Tumorzellen mit anhaftenden Immunzellen zeigen; bei Behandlung mit 12,5 µM oder 25 µM 6-PN bzw. 8-PN reduziert sich die Anzahl der vitalen Tumorzellen deutlich (rote Pfeile). **B** Die Quantitative Auswertung der Tumorzellen 48, 72 und 96 h nach Behandlungsbeginn zeigt eine signifikante Abnahme der Melanomzellen unter Behandlung mit 12,5 µM oder 25 µM 6-PN bzw. 8-PN; 3 unabhängige Zählungen in Triplikaten; One-way ANOVA Dunnetts multiple comparison test, ***P*<*0.01.*

### 6. Bestätigung der bisherigen Steigerung der NK-Zell Viabilität mit automatischem Zellzählgerät

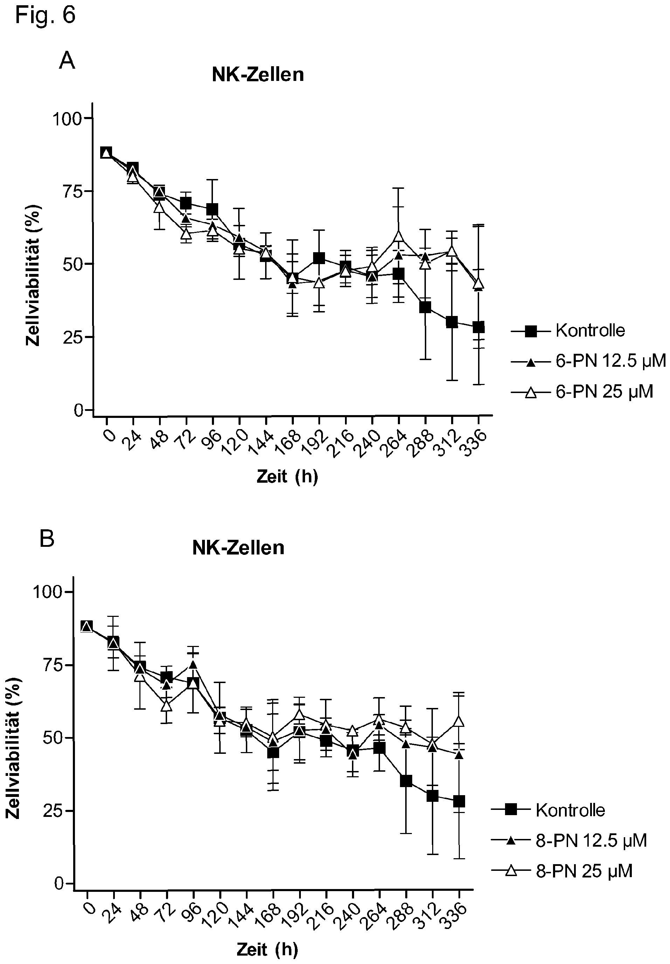

In einem Folgeexperiment wurde gezeigt, dass 6-PN und 8-PN die Viabilität von NK-Zellen von gesunden Spendern erhöhen/verlängern, gemessen in einem automatisierten Viabilitäts-/Zellzähl-Gerät. Die Untersuchung ist damit unabhängig von dem Experimentator. Das Ergebnis ist in der Fig. 6 dargestellt. NK-Zellen von zwei gesunden Spendern wurden einmalig mit 12,5 µM oder 25 µM 6-PN bzw. 8-PN behandelt und anschließend täglich zu unabhängigen Zeitpunkten mittels eines Nucleocounter NC-250 Zellzählgerätes auf Viabilität über einen Zeitraum von 336 h untersucht; gezeigt Mittelwert +/- SD.

### 7. Ausweitung der Viabilitätssteigerung auf die übergeordnete Zellpopulation: Mononukleäre Zellen des peripheren Blutes (PBMCs)

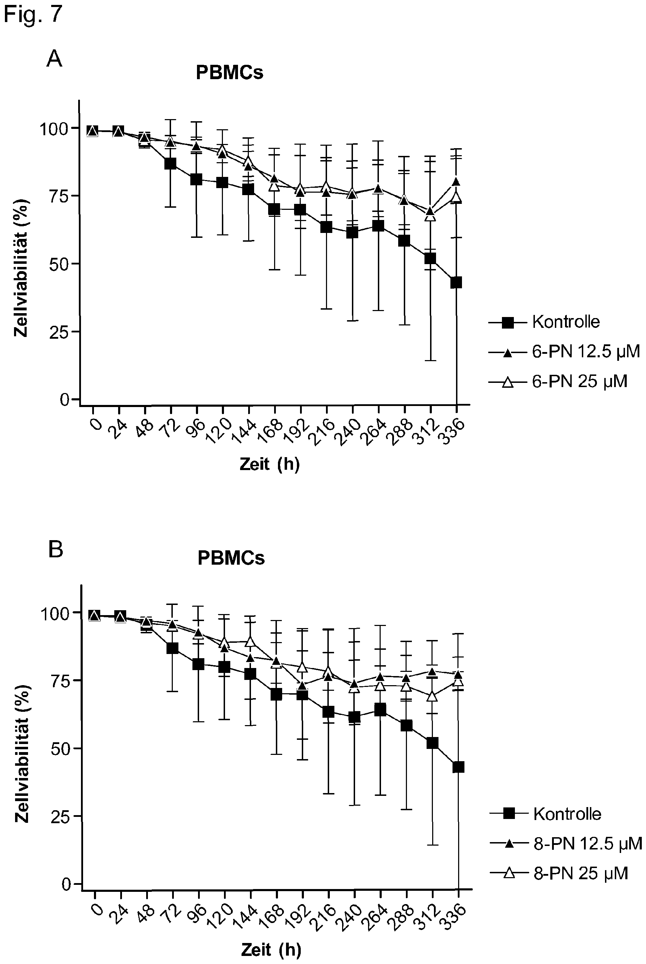

In einem weiteren Experiment wurde gezeigt, dass 6-PN und 8-PN die Viabilität von PBMCs von gesunden Spendern erhöhen/verlängern, gemessen in einem automatisierten Viabilitäts-/Zellzähl-Gerät. Das Ergebnis ist in der Fig. 7 dargestellt: PBMCs von drei gesunden Spendern wurden einmalig mit 12,5 µM oder 25 µM 6-PN bzw. 8-PN behandelt und anschließend täglich zu unabhängigen Zeitpunkten mittels eines Nucleocounter NC-250 Zellzählgerätes auf Viabilität über einen Zeitraum von 336 h untersucht; gezeigt Mittelwert +/- SD.

### 8 Bestätigung der aktivierenden Aktivität auf PBMCs mittels alternativem Assay und erneute Bestätigung mittels kommerziell erhältlicher NK-Zelllinie (NKL)

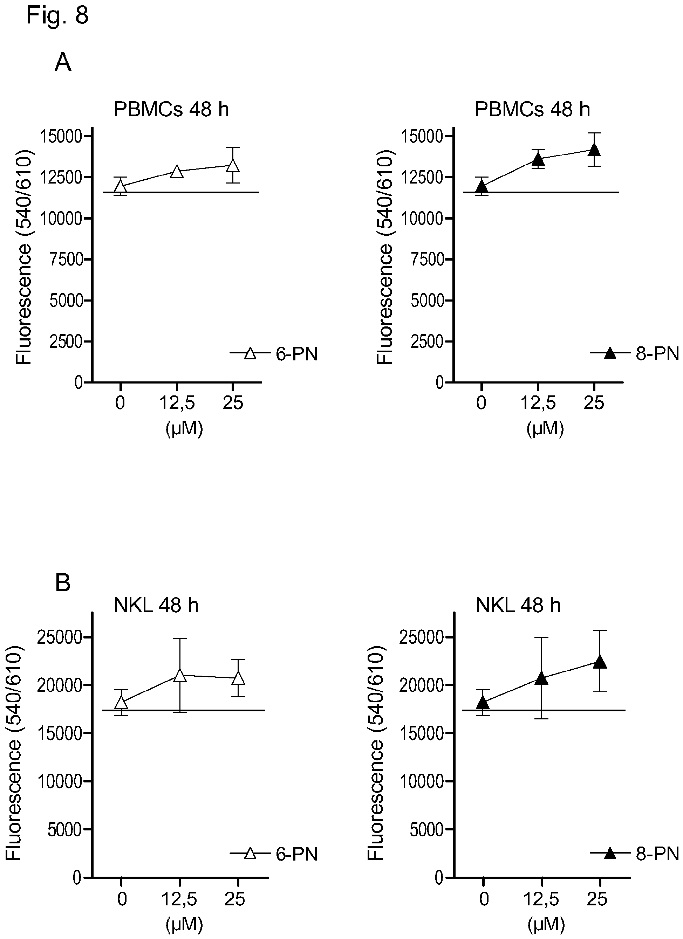

In einem anderen Experiment wurde gezeigt, dass 6-PN und 8-PN die metabolische Aktivität von PBMCs von gesunden Spendern oder der standardisierten, käuflichen NK-Zelllinie NKL erhöhen, gemessen mit dem Promega CellTiter-Blue Assay Das Ergebnis ist in der Fig. 8 gezeigt: **A** PBMCs von zwei gesunden Spendern wurden einmalig mit 12,5 µM oder 25 µM 6-PN bzw. 8-PN behandelt und nach 48 h untersucht. **B** NKL-Zellen wurden einmalig mit 12,5 µM oder 25 µM 6-PN bzw. 8-PN behandelt und nach 48 h untersucht. Bei beiden Immunzellpopulationen und bei beiden Behandlungen ist ein Anstieg der metabolischen Aktivität zu detektieren; gezeigt Mittelwert +/- SD.

### Fazit

Die Erfinder konnten demonstrieren, dass 6-Prenylnaringenin (6-PN) und 8-Prenylnaringenin (8-PN) immunstimulatorische Wirkungen auf das angeborene Immunsystem bzw. NK-Zellen ausüben. Diese natürlichen HDACi (i) erhöhen massiv die Viabilität von NK-Zellen, (ii) verlängern das Gesamtüberle ben von NK-Zellen und (iii) aktivieren NK-Zellen bzw. bewirken ein gesteigertes "Killing" von humanen entarteten bzw. infizierten Zellen durch NK-Zellen.

## Patentansprüche

1. Zusammensetzung aufweisend 6- und/oder 8-Prenylnaringenin als Wirkstoff zur Verwendung in der Immunstimulation.

2. Zusammensetzung nach Anspruch 1, zur Verwendung nach Anspruch 1, **dadurch gekennzeichnet, dass** es sich bei der Immunstimulation um eine solche des angeborenen Immunsystems handelt.

3. Zusammensetzung nach einem der vorherigen Ansprüche, zur Verwendung nach einem der vorherigen Ansprüche, **dadurch gekennzeichnet, dass** die Immunstimulation eine Stimulation und/oder Erhöhung der Viabilität und/oder der Erhöhung der Lebensdauer und/oder Erhöhung der Aktivität der Natürlichen Killerzellen (NK-Zellen) bewirkt.

4. Zusammensetzung nach einem der vorherigen Ansprüche, zur Verwendung nach einem der vorherigen Ansprüche, **dadurch gekennzeichnet, dass** es sich bei der Zusammensetzung um eine pharmazeutische Zusammensetzung handelt.

5. Zusammensetzung nach Anspruch 4 zur Verwendung nach Anspruch 4 in der Behandlung einer Krankheit, die ausgewählt ist aus der Gruppe bestehend aus: viralen Infektionen, Autoimmunerkrankungen, Tumorerkrankungen.

6. Zusammensetzung nach einem der Ansprüche 1 bis 3, zur Verwendung nach einem der Ansprüche 1 bis 3, **dadurch gekennzeichnet, dass** es sich bei der Zusammensetzung um ein Lebensmittelprodukt handelt.

7. Zusammensetzung nach Anspruch 6, zur Verwendung nach Anspruch 6, **dadurch gekennzeichnet, dass** es sich bei der Zusammensetzung um ein Lebensmittelprodukt auf Hopfenbasis handelt.

8. Zusammensetzung nach einem der vorherigen Ansprüche, zur Verwendung nach einem der vorherigen Ansprüche, **dadurch gekennzeichnet, dass** der Wirkstoff in micellierter Form vorliegt.

## Claims

1. Composition comprising 6- and/or 8-prenylnaringinin as an active agent for a use in the immunostimulation.

2. Composition of claim 1 for a use of claim 1, **characterized in that** the immunostimulation is such of the inert immune system.

3. Composition of any of the preceding claims for a use of any of the preceding claims, **characterized in that** the immunostimulation results in a stimulation and/or an increase of the viability and/or an increase of the life span and/or an increase of the activity of the natural killer cells (NK cells).

4. Composition of any of the preceding claims for a use of any of the preceding claims, **characterized in that** the composition is a pharmaceutical composition.

5. Composition of claim 4 for a use of claim 4 for the treatment of a disease which is selected from the group consisting of: viral infections, autoimmune diseases, tumor diseases.

6. Composition of any of claims 1 to 3 for a use of any of the claims 1 to 3, **characterized in that** the composition is a food product.

7. Composition of claim 6 for a use of claim 6, **characterized in that** the composition is a food product on the basis of hops.

8. Composition of any of the preceding claims for a use of any of the preceding claims, **characterized in that** the active agent is present in micelled form.

## Revendications

1. Composition présentant du 6- et/ou 8-prenylnaringenin en tant que principe actif, pour son utilisation dans l'immunostimulation.

2. Composition selon la revendication 1 pour son utilisation selon la revendication 1,
**caractérisée en ce que** l'immunostimulation est une immunostimulation du système immunitaire congénital.

3. Composition selon l'une quelconque des revendications précédentes, pour son utilisation selon l'une quelconque des revendications précédentes, **caractérisée en ce que** l'immunostimulation entraîne une stimulation et/ou une augmentation de la viabilité et/ou une augmentation de la durée de vie et/ou une augmentation de l'activité des cellules tueuses naturelles (cellules NK).

4. Composition selon l'une quelconque des revendications précédentes, pour son utilisation selon l'une quelconque des revendications précédentes, **caractérisée en ce que** la composition est une composition pharmaceutique.

5. Composition selon la revendication 4, pour son utilisation selon la revendication 4 dans le traitement d'une maladie, qui est choisie parmi le groupe constitué : d'infections virales, de maladies auto-immunes, de maladies tumorales.

6. Composition selon l'une quelconque des revendications 1 à 3, pour son utilisation selon l'une quelconque des revendications 1 à 3, **caractérisée en ce**
**que** la composition est un produit alimentaire.

7. Composition selon la revendication 6, pour son utilisation selon la revendication 6,
**caractérisée en ce que** la composition est un produit alimentaire à base de houblon.

8. Composition selon l'une quelconque des revendications précédentes, pour son utilisation selon l'une quelconque des revendications précédentes, **caractérisée en ce que** le principe actif est présent sous une forme micellaire.
